# EUROPEAN PATENT SPECIFICATION

(11) **EP 2 082 699 B1**
(45) Date of publication and mention of the grant of the patent: **09.04.2014**
(21) Application number: 09151088.3
(22) Date of filing: 22.01.2009
(51) Int. Cl.: A61B 18/08, A61B 18/22, A61B 19/00, A61B 17/00, A61B 18/00

(54) **Ablation device**
Ablationsvorrichtung
Dispositif d'ablation

(30) Priority: 25.01.2008 GB 0801419
(43) Date of publication of application: 29.07.2009
(73) Proprietor: Antheia Products Limited, Stratford upon Avon Warwickshire CV37 6JG (GB)
(72) Inventor: Finlay, Patrick, Beaconsfield, Buckinghamshire HP9 1AE (GB)
(74) Representative: Parnham, Kevin

(56) References cited:
- WO-A-00/51486
- WO-A-93/12728
- US-A- 6 032 673
- US-A1- 2002 010 502
- US-A1- 2006 270 916

## Description

THIS INVENTION relates to a surgical tool for use as an ablation device and an ablation system according to independent claim 1. More specifically, the surgical tool is preferably a cystoscope.

It is known to perform various different surgical operations on a patient utilising images of the patient which have previously been acquired by, for example, ultrasound imaging, x-rays or magnetic resonance imaging. During such operations a surgeon may be guided by a previously acquired image when selecting, for example, the quantity of bone to be removed when preparing a patient for the fitting of an artificial joint. It is also known to provide robots which, to varying extents, assist the surgeon during such operations.

However, if the part of the patient being operated on is subject to movement as a result of the operation, then previously acquired images of that part of the patient are no longer representative of the true current configuration of the part of the patient. As such, the value of the previously acquired images to the surgeon is reduced. Similarly, a robot which has been programmed with the previously acquired images may have difficulty in continuing with the operation safely without risking injury to the patient because it is no longer aware of the true configuration of the parts of the patient.

This issue is highlighted during operations to remove parts of a patient's body which do not maintain their shape or have a reduced rigidity as a result of partial removal of that part (and are, therefore, more prone to movement). Thus, although previously acquired images of the relevant part of the patient may initially be accurate, the nature of the operation means that the images will inevitably become obsolete as the operation progresses.

It is known to acquire additional images during an operation in order to update previously acquired images to provide a surgeon and/or a robot with current information regarding the configuration of the part of the patient on which an operation is being carried out. Nevertheless, in certain types of operation the inevitable change in configuration of the part of the patient on which the operation is being carried out prevents the surgeon and/or a robot from accurately identifying the configuration and location of parts of the patient which are affected by the operation.

One such operation is transurethral resection of the prostate (TURP) for the treatment of benign prostatic hyperplasia (BPH). Various different forms of TURP surgery have been developed including electrovaporisation and visual laser ablation.

During such a TURP operation a cystoscope is inserted through the urethra of a patient until the end of the cystoscope reaches the prostate. The cystoscope includes an ablation or resection device (which may be a heated wire loop or laser). The surgeon utilises the ablation or resection device to remove (i.e. resect or ablate) a portion of the prostate to relieve the restriction of the urethra caused by the BPH condition.

Figure 1 shows a schematic cross-sectional diagram depicting the bladder 1, prostate 2, and urethra 3 prior to transurethral resection of the prostate 2.
Figure 2 shows a similar schematic cross-sectional view of the bladder 1, prostate 2, and urethra 3 after a successful transurethral resection of the prostate.

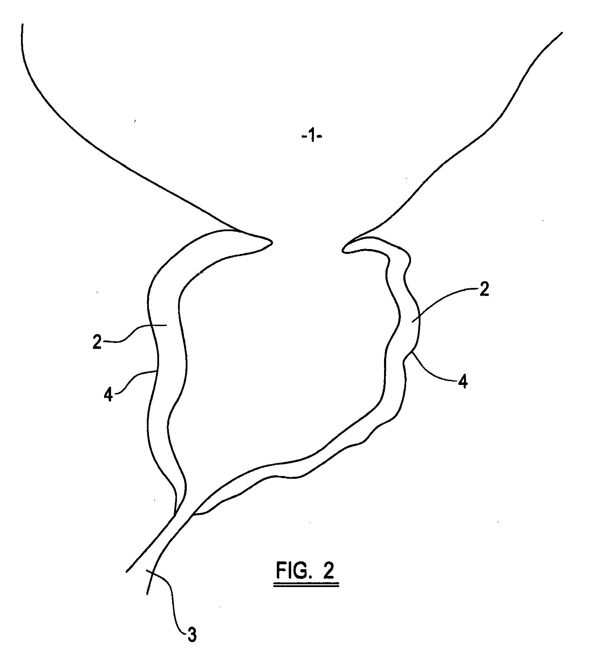

As can be seen in figure 2, a large portion of the interior of the prostate 2 is removed during the TURP surgery. A wall of the prostate 2 is retained. This wall may be between 3 and 7 mm thick (although the thickness of the wall may be dependent on various conditions and, as such, could be greater or less).

As can also be seen, the shape of the prostate 2 changes as an inevitable consequence of the resection procedure (ie. the outer peripheral surface 4 of the prostate 2 moves as prostate material is removed). As such, it is often difficult for a surgeon to determine the precise amount of prostate material which has been removed. Moreover, the substantial change in the shape of the prostate 2 means that it is extremely difficult (if not impossible) for a completely automated process to be implemented utilising a robot. Specifically, because the shape of the remaining prostate 2 changes during the operation, it is difficult for the robot to keep track of the thickness of the remaining prostate material and to ensure that the patient is not caused any injury.

US2007/0233045 is a US patent application which discloses an intravascular catheter which includes a fluid delivery system which is configured to inflate one or more balloons along a telescopic arm of the catheter. Through a sequence of inflation and deflation, extension and contraction steps, the catheter is able to move along a vessel. The catheter can have a cutting tool attached to an end thereof or, alternatively, the catheter may be open at the end to allow a drug to be delivered to a patient.

WO 93/12728 discloses an ablation module in which the rate of fluid flow may be adjusted to produce the desired distention of the uterus.

It is an object of the present invention to seek to ameliorate the problems associated with the prior art.

Accordingly, one aspect of the present invention provides an ablation device as claimed in claim 1. Dependent features are defined in the dependent claims.

Another aspect of the present invention provides an ablation system comprising: an ablation device according to claim 1 and any dependent claim and a robot configured to control movement of the surgical tool.

Preferably, the robot is configured to drive movement of the surgical tool in accordance with a predetermined set of instructions.

Conveniently, the robot is configured to limit the movement of the surgical tool in accordance with a predetermined set of instructions.

Advantageously, the predetermined instructions are updated upon receipt by the robot of a feedback signal.

Conveniently, the robot is configured to control the movement of the surgical tool in three mutually orthogonal axes.

Preferably, the axes provide one translational and two rotational degrees of freedom of movement.

Advantageously, the axes are confocal on a predetermined location.

Preferably, the system further comprises a sensing device configured to sense at least one of the size, position, and thickness of at least part of the surgical site.

Conveniently, the sensing device is configured to sense at least one of the size, position, and thickness of at least part of the surgical site during a surgical operation.

Advantageously, information from the sensing device is utilised to update a set of instructions of the robot which control the movement of the surgical tool.

Preferably, the information from the sensing device is utilised to increase or decrease the pressure of fluid delivered to the surgical site.

Conveniently, the sensing device is configured to be inserted into a natural orifice of a human.

Advantageously, the sensing device is attached to the surgical tool.

Another aspect of the present invention provides, an ablation system comprising: an ablation device according to claim 1 in a surgical operation and a robot configured to control movement of the surgical tool, wherein the surgical tool comprises: an ablation module output configured to ablate a volume of material at a surgical site to form an ablated volume; and a fluid output adapted to deliver fluid to the surgical site during a surgical operation such that at least part of the ablated volume is filled with fluid and the fluid is in direct contact with at least part of the surgical site.

Conveniently, receiving information comprises receiving information which is size or position information for an organ of which the surgical site is a part.

Embodiments of the present disclosure will now be described, by way of example, with reference to the accompanying drawings in which:

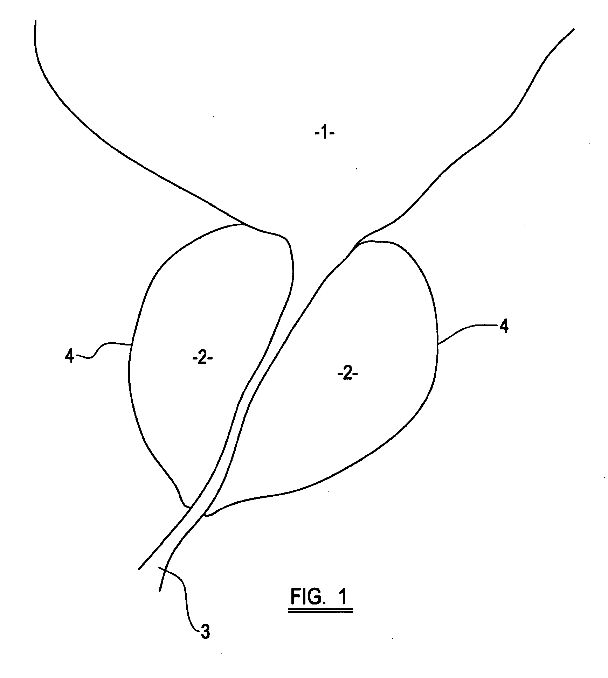

Figure 1 shows a schematic cross-sectional view of a prostate;
Figure 2 shows a schematic cross-sectional view of a prostate following a transurethral resection procedure;

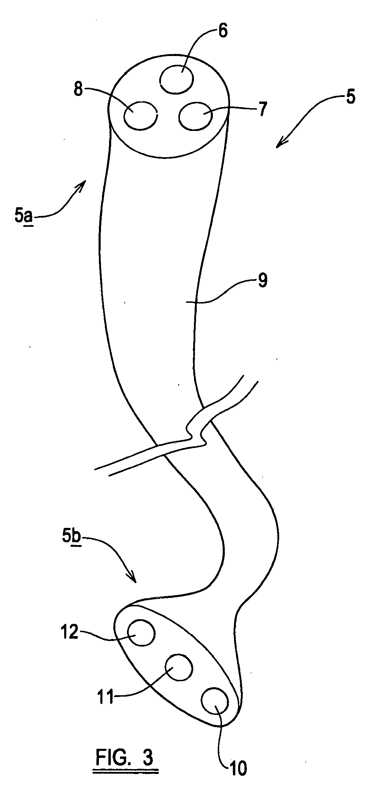

Figure 3 shows a schematic representation of an ablation device in accordance with an embodiment of the present disclosure;

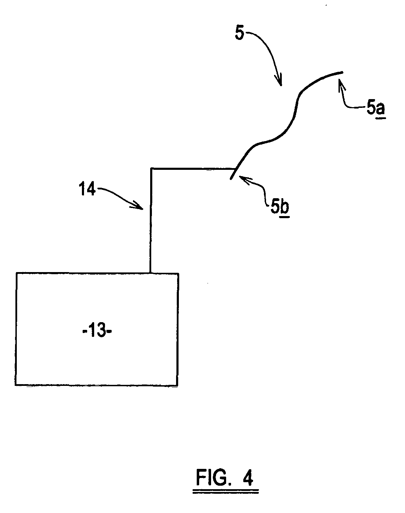

Figure 4 shows a schematic representation of a robot including an ablation device in accordance with the present invention;

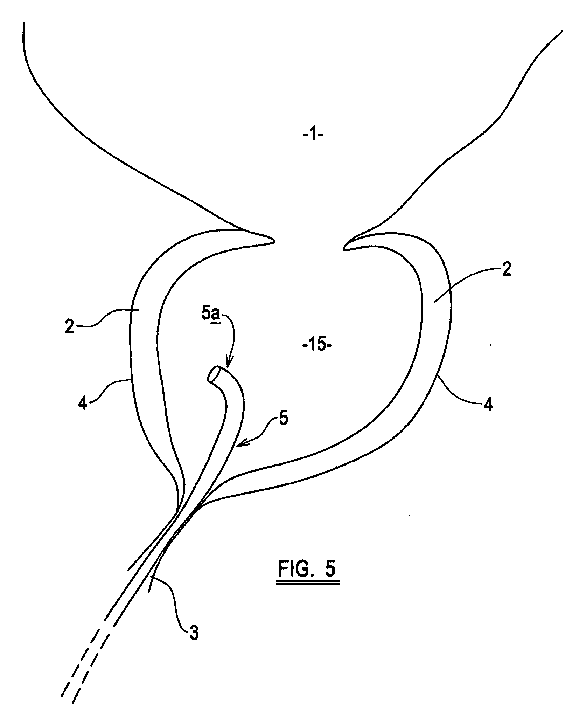

Figure 5 shows a schematic cross-sectional view of a prostate and ablation device in accordance with an embodiment of the present invention;

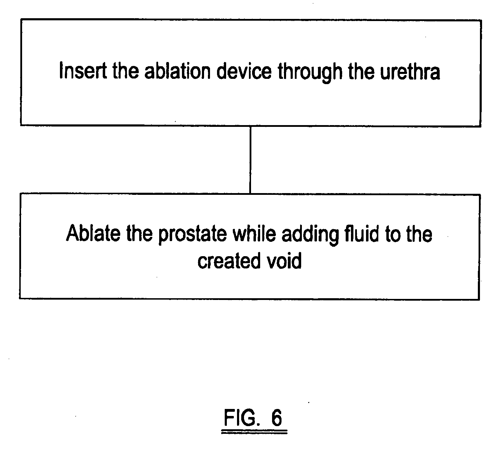

Figure 6 shows a flowchart depicting a method utilising an ablation device in accordance with an embodiment of the present disclosure;

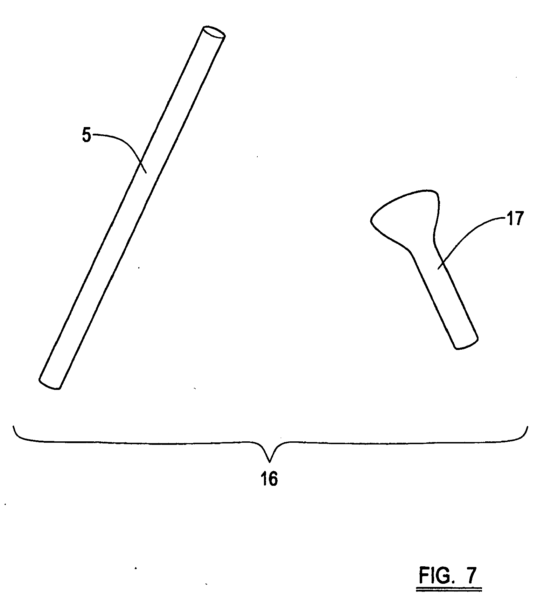

Figure 7 shows an ablation system in accordance with an embodiment of the present disclosure; and

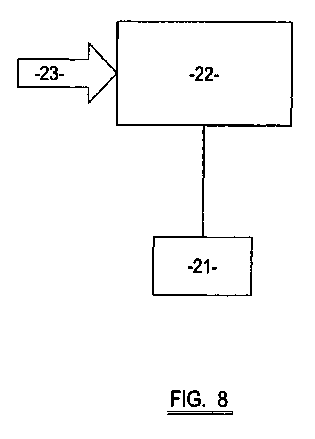

Figure 8 shows a feedback system of the present disclosure.

An ablation device 5 in accordance with an embodiment of the present disclosure is a surgical tool and is depicted in figure 3. The device 5 is preferably a cystoscope (or a part of a cystoscope) and comprises an elongate body 9 having a proximal end 5b, a distal end 5a.

At the distal end 5a of the device 5 there is an optical input 8 (which may be omitted in some embodiments), a light source (not shown - and which may be omitted in some embodiments) an ablation module output 6, and a fluid output 7. At the proximal end 5b of the device 5 there is an optical output 10 (which may be omitted in some embodiments), an ablation module input 11, and a fluid input 12.

The optical input 8 preferably comprises a lens which is optically coupled to a telescope (not shown) which runs the length of the elongate body 9 of the device 5. The telescope is optically coupled at the proximal end 5a of the device 5 to the optical output 10.

The optical output 10 may comprise a lens (not shown) through which a surgeon may look or to which a camera (not shown) may be connected. If a camera is connected to the lens, then images captured by the camera may be displayed on a display screen (not shown). This allows a surgeon to see an image of the patient visible to the distal end 5a of the device 5. The optical input 8, optical output 10 and telescope comprise an optical system of the device 5. A light source may be provided in order to illuminate the surgical site at the distal end 5a of the device 5.

The fluid output 7 is coupled to a fluid transportation conduit (not shown) which runs the length of the elongate body 9 of the device 5 and is coupled at the proximal end 5a of the device 5 to the fluid input 12. The fluid conduit, fluid output 7, and fluid input 12 are configured to permit a fluid to be input into the fluid input 12, passed through the fluid conduit and output of the device 5 through the fluid output 7 (and thus form the parts of a fluid delivery system).

A valve 21 (see figure 8) may be present in the fluid conduit, and/or at the fluid input 12 and/or at the fluid output 7, to allow the flow of fluid to occur in substantially only a first direction (from the input 12 to the output 7). The or each valve 21 may be configured to be actuated to allow the flow of fluid in a second direction (which opposes the first direction). The or each valve 21 may be a configured to control the rate of flow of fluid in either the first or second direction. To this end, the or each valve 21 may define a valve opening (not shown) with a variable size. If the or each valve 21 is controlled to have a relatively small valve opening, then the rate of flow of fluid through the valve 21 is comparatively low. On the other hand, if the or each valve 21 is controlled to have a relatively large valve opening, then the rate of flow of fluid through the valve 21 is comparatively high. Thus, by controlling the size of the or each valve opening it is possible to control the rate of flow of fluid through the device 5.

The or each valve 21 may be formed from several valves (not shown) in series. Each valve in a series of such valves may perform a different function (e.g. controlling the direction of flow of fluid through the valve or the rate of flow of fluid through the valve) and/or be individually controllable. This arrangement has the potential to permit a high level of control over the direction and rate of flow of fluid through the device 5.

The or each valve 21 may be configured to control the flow of fluid therethrough in a stepped manner (i.e. through a discrete number of different flow rates/pressures). Alternatively, the or each valve 21 may be configured to control the flow of fluid in a continuous manner over a range - the range may be from a completely closed state (in which no fluid flows through the valve) to a completely open state (in which there is substantially no obstruction to the flow of fluid through the valve).

The ablation module output 6 is coupled to an ablation module control channel (not shown) which runs the length of the elongate body 9 of the device 5 and is coupled at the proximal end 5a of the device 5 to the ablation module input 11.

The ablation module output 6 is the output from an ablation module (not shown) which may be a laser. If the ablation module is a laser, then the laser may be a front or side-firing laser. The ablation module may be located in the device 5 or may be remote from the device 5. Alternatively, the ablation module may comprise a heated wire (not shown) or a sharp cutting tool (not shown).

The ablation module control channel may be an optical channel adapted to transmit a laser from the ablation module input 11 to the ablation module output 8. Alternatively, the control channel may comprise a channel suitable to transmit a control signal to cause activation and/or deactivation of ablation module in the device 5. As such, the ablation module is coupled to both the ablation module input 8 and the ablation module output 11.

The ablation module may be located at the ablation module output 6 or at the ablation module input 11.

The elongate body 9 is preferably substantially rigid.

Alternatively, the elongate body 9 of the device 5 is at least partially flexible and of a sufficient length to be passed through the urethra 3 of a human to the prostate 2 such that the proximal end 5a of the device 5 extends out of the urethra of the patient and the distal end 5b of the device is located generally at the prostate 2 of the patient.

If the device 5 is flexible, then a control system may be provided to control the flexing of the device 5 in a desired manner so that the device can be navigated through the patient's urethra.

An embodiment of aspects of the present invention may include additional features such as that the elongate body 9 of the device 5 includes an attachment arrangement (not shown) at the proximal end 5a. The attachment arrangement is operable to permit the device 5 to be coupled to a further elongate body (not shown) which has a length such that when the device 5 and further elongate body are coupled together, the distal end 5b of the device 5 may be located at the prostate 2 of a human patient and a free end of the further elongate body extends out of the urethra 3 of the patient. In this embodiment with additional features the elongate body 9 of the device 5 may be substantially rigid.

The ablation device 5 may be coupled to a robot 13. Preferably, a supporting arm 14 of the robot 13 holds the proximal end 5b of the device 5 and includes a control module (not shown) to control movement of the ablation device 5. The control module may include one or more motors to drive the ablation device 5 in pan, tilt and zoom movements with respect to the prostate 2 (preferably, these axes of movement are mutually orthogonal and share a common point of axial intersection which is located between the open end of the urethra 3 and the prostate 2). Preferably, the robot 13 is configured to drive the ablation device in one translational and two rotational degrees of freedom of movement. Advantageously, the axes are confocal - for example, on an orifice of the patient or other predetermined location.

An embodiment of aspects of the present invention may include additional features such as that the control module does not drive movement of the ablation device 5 but is configured to resist movement of the ablation device 5 in certain circumstances (as will be explained below).

In an ablation system 16 in accordance with an embodiment of the present invention the system combines (see figure 7) the ablation device 5 with the sensing device 17. The sensing device 17 is preferably an ultrasound transceiver which is configured to utilise ultrasound signals to measure the size, thickness and/or position of certain parts of the human body which may be the subject of a surgical operation - for example a prostate 2.

The sensing device 17 is advantageously configured to be inserted into a natural orifice of the patient (for example, their rectum) and sense the position and size of at least part of the prostate 2. An artificial orifice may also be used in the form of an incision.

The sensing device 17 may form part of the ablation device 5 and may be located adjacent the ablation module output 6. The ablation module output 6 and the sensing device 17 may be operated alternately - in other words, when the ablation module output 6 is actuated to ablate material the sensing device 17 is turned off, and vice versa. This may help to protect the sensing device 17 from damage and/or increase the accuracy of any measurements made by the sensing device 17. A control element (not shown) may be provided to actuate the sensing device 17 and the ablation module output 6 alternately in a repeated fashion. This repeated actuation may be at a relatively low frequency (eg. actuation occurring every few minutes) or may be at a sufficiently high frequency (eg. in the order of several hertz) that substantially constant operation of both the ablation module output 6 and the sensing device 17 is experienced by an operator.

The sensing device 17 may be coupled to the robot 13.

The ablation system 16 may further comprise the above described robot 13.

The ablation system 16 may further include an external sensing device (not shown) which is configured to sense the location and size of the prostate 2. The external sensing device may be configured for insertion into a natural orifice of the patient (for example, their rectum) but is preferably adapted for external use.

The external sensing device may be an x-ray device, an MRI device or an ultrasound device - for example.

The external sensing device and the sensing device 17 may be one and the same device.

The external sensing device may be coupled to the robot 13 and configured to pass information to the robot 13 concerning the size, location and/or thickness of the prostate 2 (for example).

In operation, a patient is presented requiring surgical resection of a part of their anatomy which will not remain In a fixed configuration throughout the procedure. For example, the patient may require a TURP-type operation.

In this example, the external sensing device is utilised to obtain the size and location of the prostate 2. This information is input to the robot 13 and presented to a surgeon (or other operator) who may select the area of the prostate 2 which is to be removed. This function may be preformed by the surgeon utilising a visual interface of the robot 13 (for example).

In an embodiment in accordance with aspects of the present invention, the surgeon may additionally and initially activate a computer program stored on the robot 13 which automatically selects a region of the prostate 2 to be removed with the surgeon adjusting the ablation device relative to the computer program so leaving behind a wall of prostate material 2 which has a predetermined thickness (for example, 3mm to 7mm). Alternatively, the surgeon may input the desired thickness of prostate material 2 which is to be retained and the robot 13 may store this value for future reference.

Preferably, the surgeon inputs to the robot 13 (or the robot 13 calculates from the above information) a set of one or more instructions to control movement of the ablation device 5.

The sensing device 17 is preferably inserted into the patient's rectum to a location at which the device 17 can monitor the size, position and/or thickness of the prostate 2.

The ablation device 5 is inserted into the patient's urethra 3 until the distal end 5a thereof is located substantially with the prostate 2. This may be achieved by using information obtained from the external sensing device to ensure that the distal end 5a of the device 5 is at the correct location. Alternatively, the surgeon may position the ablation device 5 manually by utilising the image presented at the optical output 10 of the device 5. Alternatively, the sensing device 17 may be configured to identify the location of the distal end 5a of the device 5 such that the distal end 5a of the device can be correctly located at the patient's prostate 2. Alternatively, a combination of these methods is utilised.

Fluid is delivered to the fluid input 12 and output through the fluid output 7. The fluid at least partially fills the area of the urethra in the region of the prostate 2.

The ablation module is activated. Activation of the ablation module may be an automatic event controlled by the robot 13 or may be controlled by the surgeon. If the ablation module output 6 is coupled to a laser, then activation causes the laser beam to be output by the ablation module output 6 to ablate the prostate material 2 at the distal end 5a of the device 5.

The device 5 is moved with respect to the prostate 2 to cause prostate material 2 to be ablated in the desired manner. As this ablation process is occurring fluid (from the fluid output 7) enters the region of the prostate 2 which was previously occupied by prostate material 2. This causes the prostate 2 substantially to retain Its shape during the operation. As such, the information gathered by the external sensing device remains substantially accurate. The fluid entry may be continuous, substantially continuous, periodic or otherwise controlled (by a valve 21 for example). This control is automatic (ie. controlled by the robot 13) but this automatic control may be adjusted and controlled manually by the surgeon. It will be appreciated that the fluid which enters the region of the prostate 2 which was previously occupied by prostate material 2 must be under sufficient pressure to maintain a boundary of the prostate 2 in a substantially constant position and/or to maintain the prostate 2 such that it retains a substantially constant size (i.e. the rate of flow of fluid into the prostate 2 must be sufficient to maintain a boundary of the prostate 2 in a substantially constant position and/or to maintain the prostate 2 such that it retains a substantially constant size).

If the above process is automatic, then the automatic control is such that a higher pressure of fluid (i.e. fluid at a higher flow rate) is output into the ablated prostate 2 volume if the prostate reduces in size and, similarly, a lower pressure of fluid (i.e. fluid at a lower flow rate) is output into the ablated prostate 2 volume if the prostate increases in size (the reduction or increase in size is preferably relative to a size of the prostate 2 sensed by the sensing device 17 or external sensing device in a pre-operative configuration). If a manual process is utillised then the operator will aim to achieve the same effect as the automatic process by varying the pressure of the fluid.

In an embodiment utilising automatic control of the rate of fluid flow into the prostate 2, a control module 22 may be provided (see figure 8). The control module 22 receives information 23 from one or more sensors which may include the sensing device 17 or the external sensing device. This information 23 includes at least the relative size and position of the prostate 2 when compared to the size and position of the prostate 2 captured prior to the procedure using the external sensing device. Preferably, absolute prostate 2 size and position information 23 is received by the control module 22.

The control module 22 uses the information 23 which has been received from the or each sensor to determine if the boundary of the prostate 2 has changed position or shape. If a change is detected then the control module 22 controls the or each valve 21 to alter the rate of flow of fluid to the prostate 2. For example, if the boundary of the prostate 2 is found to have decreased in size, then the control module 22 may actuate the or each valve 21 in order to increase the pressure (i.e. rate of flow) of fluid entering the prostrate 2 so that the boundary of the prostate 2 increases in size - to maintain the accuracy of the original images of the prostate as obtained by the external sensing device. Similarly, the pressure of fluid may be reduced if the boundary of the prostate is found to be greater in size than the boundary as originally determined by the external sensing device. In other words, a feedback system is provided which monitors the size and position of the prostate 2 and controls the operation of the or each valve 21 in response.

If the position of the prostate 2 has been determined (by the control module 22) to have changed, and the discrepancy cannot be corrected by adjustment of the or each valve 21, then the control module 22 may adjust the position of the device 5 - if the ablation device 5 is under robotic control. In any event, a notification of the change in position may be output by the control module 5 for the attention of, for example, a surgeon. The notification may include a suggested adjustment of the position of the ablation device 5 which the surgeon can comply with manually, accept as a movement to be carried out under robotic control, or reject (for example, as inappropriate).

It will be appreciated that the information 23 regarding the size and position of the prostrate 2 which is received by the control module 22 during an operation may not be as accurate or complete as the size and position information which was originally determined by using information from the external sensing device before the operation. As such, the control module 22 may be programmed to ignore small discrepancies - in other words, it may be that the control module 22 must determine that a disparity between the current position and/or size and the predetermined position and/or size is greater than a threshold value before action will be taken.

It will be appreciated that the control module 22 may need to extrapolate information 23 which is received during an operation in order to provide a prediction of the current size and position of the prostate 2. For example, the level of information 23 received by the control module 22 during an operation may not be as complete as the level of information which was acquired prior to the operation. The control module 22 is preferably configured to receive this limited information 23 (which may include, for example, the distance of part of the prostate 2 from the sensing device 17) and use this information 23 to predict the location and/or size of the prostate 2 for comparison with the originally acquired information (i.e. the predetermined information).

Accordingly, movement of the ablation device 5 can be entirely or partially automated. For example, the robot 13 may move the ablation device 5 to cause ablation of the prostate 2 in the desired manner (for example, to leave a wall of prostate material 2 approximately 3mm - 7mm thick); alternatively, the robot 13 may allow the surgeon to drive movement of the ablation device 5 but may restrict movement of the device 5 which would cause excessive ablation of a region of the prostate 2 (for example, to leave a section of the wall of the prostate 2 with a thickness which is not between 3mm and 7mm).

There may be a quantity of fluid leakage from the region of the prostate 2 during the ablation which may cause the actual size and position of the prostate 2 to vary from the information acquired by the external sensing device and this may cause small errors in the operation of the robot 13. As such, the rate of fluid input through the fluid input 7 is preferably substantially equal to or greater than the fluid leakage rate.

The sensing device 17 may be utilised to monitor the size, location and/or thickness of the prostate 2 during the operation and to identify any deviations of this information from the information previously acquired by the external sensing device. In the event of a disparity, the sensing device 17 may update the information provided to the robot 13 with actual current location and size information (for example, by the issuance of a feedback signal). The sensing device 17 may instruct the rate of fluid input into the region of the prostate 2 to be increased or decreased to correct the disparity. The sensing device 17 may raise an error and cause deactivation of the ablation device 5. The sensing device 17 may cause the valve of the fluid conduit, fluid output 7 or fluid input 12 to actuate and cause the fluid to be evacuated from the region of the prostate 2. The sensing device 17 may alter a set of instructions of the robot with current information regarding the prostate 2.

The sensing device 17 and/or the external sensing device may be utilised in order to register the location of the volume in relation to which the ablation device 5 is being utilised. In particular, in order to utilise fully-automatic operation of the ablation device 5 or even some levels of limited automation, it is necessary to register the location of at least part of the prostate 2 (for example) and the ablation module 5 within a common frame of reference. In addition, if any pre-operative information (such as images) has been obtained (for example by the external sensing device) then this information may also be registered in the common frame of reference. This may be achieved by utilising the sensing device 17 and/or the external sensing device. For example, the location of the ablation device 5 can be sensed (using, for example, the sensing device 17 or external sensing device), or the location of the ablation device 5 can be determined using a known length and orientation of the elongate body of the device in combination with a location of known position with respect to the sensing device 17 and/or external sensing device. Accordingly, the location of the distal end 5a of the ablation device 5 can be determined with respect to the prostate 2 (as sensed by the sensing device 17 and/or external sensing device) and movement of the ablation device controlled accordingly, for example, to ablate a region of the prostate 2.

The above method is summarised in figure 6.

Use of the sensing device 17 is not considered to be essential.

It will be appreciated that monitoring the position and/or size of the prostate 2 during an operation ensures that the position of the surgical site within the prostate 2 (i.e. the site of the actual surgery) is monitored.

The specific fluid which is utilised will depend upon the particular operation being performed but suitable fluids may include air, nitrogen, water, and saline solution. If the sensing device 17 uses ultrasound signals, then it may be necessary to provide a fluid to ensure ultrasound coupling and accurate sensing.

Although ablation has been discussed above, it will be appreciated that the use of this term is intended to have the same meaning as the term resection.

Accordingly, it will be appreciated that ablation/resection operation carried out on parts of patient (such as the prostate 2) can be fully or partially automated by using the above technique.

It will be appreciated that the embodiments of the present invention are configured to operate on a surgical site (e.g. the prostate) and to deliver fluid to the surgical site such that at least some of the movement of the surgical site is compensated by the fluid. To this end, the fluid is in direct contact with at least part of the surgical site - for example, the fluid may be in direct contact with tissue at the surgical site.

When used in this specification and claims, the terms "comprises" and "comprising" and variations thereof mean that the specified features, steps or integers are included. The terms are not to be interpreted to exclude the presence of other features, steps or components.

## Claims

1. An ablation device (5) for use in a surgical operation comprising:
- an ablation module output (6) configured to ablate in use a volume of material at a site to form an ablated volume, the ablation module output being housed in an elongated body;
- a control module (22) to control a valve arrangement (21) dependent upon information from a sensing device (17) indicative of the position of the site and;
- a fluid output (7) adapted to deliver fluid to the site In use, such that at least part of the ablated volume is filled with fluid and the fluid is in direct contact with at least part of the site, wherein the fluid output is housed in the elongate body and the control module is operable to control the valve arrangement configuration, to allow control of the flow rate of fluid delivered by the fluid output and the flow direction, between a first direction and an opposite second direction, to control the position of the site, dependent on the information.

2. A device according to claim 1 comprising
an optical system (8, 10) adapted to provide a user with a view of at least part of the site.

3. A device according to claim 1 or claim 2 adapted such that
It may be at least partially inserted into a natural orifice of a human or a human urethra (3).

4. A device according to any of claims 1 to 3 wherein the control module (22) is operable to control the flow rate of fluid delivered by the fluid output (7) to control the relative position of the site compared to a predetermined position.

5. A device according to any preceding claim, wherein information indicative of the position of the site is size or position information for an organ (4) which the site is a part is provided to the control module.

6. An ablation system comprising: a device according to any preceding claim and a robot (13) configured to control movement of the device.

7. A system according to claim 6, wherein the robot (13) is configured to drive movement of the device in accordance with a predetermined set of instructions or to limit the movement of the device in accordance with a predetermined set of instructions.

8. A system according to claim 7, wherein the predetermined instructions are updated upon receipt by the robot (13) of a feedback signal.

9. A system according to claim 6, 7 or 8, wherein the robot (13) is configured to control the movement of the device in three mutually orthogonal axes, or configured to control movement of the device in three mutually orthogonal axes which provide one translational and two rotational degrees of freedom of movement, or configured to control the movement of the device in three mutually orthogonal axes which are confocal on a predetermined location, or configured to control movement of the device in three mutually orthogonal axes which provide one translational and two rotational degrees of freedom of movement which are confocal on a predetermined location.

10. A system according to any one of claims 6 to 9, further comprising a sensing device configured to sense at least one of the size, position, and thickness of at least part of the site, or a sensing device (17) which is configured to sense at least one of the size, position, and thickness of at least part of the site during a surgical operation.

11. A system according to claim 10, wherein information from the sensing device (17) is utilised to update a set of instructions of the robot (13) which control the movement of the device.

12. A system according to claim 10 or 11, wherein the information from the sensing device (17) is utilised to increase or decrease the pressure of fluid delivered to the site.

13. A system according to any one of claims 10 to 12, wherein the sensing device (17) is configured to be inserted into a natural orifice of a human.

14. A system according to any one of claims 10 to 13, wherein the sensing device (17) is attached to the device.

## Patentansprüche

1. Ablationsvorrichtung (5) zur Verwendung bei einem chirurgischen Eingriff, umfassend:
- einen Ablationsmodulausgang (6), der so eingerichtet ist, dass er während des Gebrauchs ein Volumen von Material an einer Stelle abträgt, um ein abgetragenes Volumen zu bilden, wobei der Ablationsmodulausgang in einem länglichen Körper untergebracht ist;
- ein Steuermodul (22) zur Steuerung einer Ventilanordnung (21) in Abhängigkeit von Informationen von einer Erfassungsvorrichtung (17), die die Lage der Stelle anzeigen; und
- einen Fluidausgang (7), der so ausgelegt ist, dass er während des Gebrauchs Fluid an die Stelle abgibt, sodass zumindest ein Teil des abgetragenen Volumens mit Fluid gefüllt ist und das Fluid in direktem Kontakt zu mindestens einem Teil der Stelle steht, wobei der Fluidausgang in dem länglichen Körper untergebracht ist und das Steuermodul so betreibbar ist, dass es die Ventilanordnungskonfiguration steuert, um die Steuerung des Durchflusses von Fluid, das von dem Fluidausgang abgegeben wird, und der Fließrichtung zwischen einer ersten Richtung und einer entgegengesetzten zweiten Richtung zu ermöglichen, um die Lage der Stelle in Abhängigkeit von den Informationen zu steuern.

2. Vorrichtung nach Anspruch 1, die ein optisches System (8, 10) umfasst, das so ausgelegt ist, dass ein Benutzer zumindest einen Teil der Stelle sieht.

3. Vorrichtung nach Anspruch 1 oder Anspruch 2, die so ausgelegt ist, dass sie zumindest teilweise in eine natürliche Öffnung eines Menschen oder eine menschliche Harnröhre (3) eingeführt werden kann.

4. Vorrichtung nach einem der Ansprüche 1 bis 3, wobei das Steuermodul (22) so betreibbar ist, dass es den Durchfluss von Fluid steuert, das von dem Fluidausgang (7) abgegeben wird, um die relative Lage der Stelle im Vergleich zu einer vorher festgelegten Lage zu steuern.

5. Vorrichtung nach einem vorhergehenden Anspruch, wobei es sich bei Informationen, die die Lage der Stelle anzeigen, um Größen- oder Lageangaben für ein Organ (4) handelt, von dem die Stelle ein Teil ist, die dem Steuermodul zur Verfügung gestellt werden.

6. Ablationssystem, umfassend: eine Vorrichtung nach einem vorhergehenden Anspruch und einen Roboter (13), der so eingerichtet ist, dass er die Bewegung der Vorrichtung steuert.

7. System nach Anspruch 6, wobei der Roboter (13) so eingerichtet ist, dass er die Bewegung der Vorrichtung gemäß einer vorher festgelegten Reihe von Anweisungen lenkt oder die Bewegung der Vorrichtung gemäß einer vorher festgelegten Reihe von Anweisungen einschränkt.

8. System nach Anspruch 7, wobei die vorher festgelegten Anweisungen aktualisiert werden, wenn der Roboter (13) ein Rückkopplungssignal empfängt.

9. System nach Anspruch 6, 7 oder 8, wobei der Roboter (13) so eingerichtet ist, dass er die Bewegung der Vorrichtung in drei zueinander orthogonalen Achsen steuert, oder so eingerichtet ist, dass er die Bewegung der Vorrichtung in drei zueinander orthogonalen Achsen steuert, die einen Translations- und zwei Rotations-Bewegungsfreiheitsgrade bereitstellen, oder so eingerichtet ist, dass er die Bewegung der Vorrichtung in drei zueinander orthogonalen Achsen steuert, die auf eine vorher festgelegte Stelle konfokal sind, oder so eingerichtet ist, dass er die Bewegung der Vorrichtung in drei zueinander orthogonalen Achsen steuert, die einen Translations- und zwei Rotations-Bewegungsfreiheitsgrade bereitstellen, die auf eine vorher festgelegte Stelle konfokal sind.

10. System nach einem der Ansprüche 6 bis 9, ferner umfassend eine Erfassungsvorrichtung, die so eingerichtet ist, dass sie die Größe, Lage und/oder Dicke von zumindest einem Teil der Stelle erfasst, oder eine Erfassungsvorrichtung (17), die so eingerichtet ist, dass sie die Größe, Lage und/oder Dicke von zumindest einem Teil der Stelle während eines chirurgischen Eingriffs erfasst.

11. System nach Anspruch 10, wobei Informationen von der Erfassungsvorrichtung (17) zum Aktualisieren einer Reihe von Anweisungen für den Roboter (13) verwendet werden, mit denen die Bewegung der Vorrichtung gesteuert wird.

12. System nach Anspruch 10 oder 11, wobei die Informationen von der Erfassungsvorrichtung (17) zum Erhöhen oder Verringern des Drucks von an die Stelle abgegebenen Fluids verwendet werden.

13. System nach einem der Ansprüche 10 bis 12, wobei die Erfassungsvorrichtung (17) so eingerichtet ist, dass sie in eine natürliche Öffnung eines Menschen eingeführt wird.

14. System nach einem der Ansprüche 10 bis 13, wobei die Erfassungsvorrichtung (17) an der Vorrichtung angebracht ist.

## Revendications

1. Dispositif (5) d'ablation destiné à être utilisé durant une intervention chirurgicale comportant :
- une sortie (6) de module d'ablation configurée pour l'ablation, pendant l'emploi, d'un volume de matière au niveau d'un site de manière à former un volume de matière enlevée, la sortie du module d'ablation étant logée dans un corps allongé ;
- un module (22) de commande pour commander un agencement (21) de clapet en fonction des informations provenant d'un dispositif (17) de détection indiquant la position du site et ;
- une sortie (7) de fluide conçue pour administrer un fluide au site, pendant l'emploi, de sorte qu'au moins une partie du volume de matière enlevée est remplie de fluide et le fluide vient en contact direct avec au moins une partie du site, dans lequel la sortie de fluide est logée dans le corps allongé et le module de commande sert à commander la configuration de l'agencement de clapet, afin de pouvoir commander le débit d'écoulement du fluide administré par la sortie de fluide et la direction de l'écoulement, entre une première direction et une seconde direction opposée, afin de commander la position du site, en fonction des informations.

2. Dispositif selon la revendication 1 comportant
un système (8, 10) optique conçu pour fournir à un utilisateur une vue d'au moins une partie du site.

3. Dispositif selon la revendication 1 ou la revendication 2 conçu de façon à
pouvoir être au moins partiellement introduit dans un orifice naturel d'un sujet humain ou d'un urètre (3) humain.

4. Dispositif selon l'une quelconque des revendications 1 à 3 dans lequel le module de commande (22) sert à commander le débit d'écoulement du fluide administré par la sortie (7) de fluide afin de commander la position relative du site par rapport à une position prédéterminée.

5. Dispositif selon l'une quelconque des revendications précédentes, dans lequel des informations indiquant la position du site sont des informations concernant la taille ou la position pour un organe (4) dont le site fait partie, qui sont fournies au module de commande.

6. Système d'ablation comportant : un dispositif selon l'une quelconque des revendications précédentes et un robot (13) configuré pour commander le mouvement du dispositif.

7. Système selon la revendication 6, dans lequel le robot (13) est configuré pour entraîner le mouvement du dispositif selon un ensemble prédéterminé d'instructions ou pour limiter le mouvement du dispositif selon un ensemble prédéterminé d'instructions.

8. Système selon la revendication 7, dans lequel les instructions prédéterminées sont mises à jour au moment de la réception par le robot (13) d'un signal de rétroaction.

9. Système selon la revendication 6, 7 ou 8, dans lequel le robot (13) est configuré pour commander le mouvement du dispositif selon trois axes mutuellement orthogonaux, ou configuré pour commander le mouvement du dispositif selon trois axes mutuellement orthogonaux qui fournissent un degré translationnel et deux degrés rotationnels de liberté de mouvement, ou configuré pour commander le mouvement du dispositif selon trois axes mutuellement orthogonaux qui sont confocaux sur un emplacement prédéterminé, ou configuré pour commander le mouvement du dispositif selon trois axes mutuellement orthogonaux qui fournissent un degré translationnel et deux degrés rotationnels de liberté de mouvement qui sont confocaux sur un emplacement prédéterminé.

10. Système selon l'une quelconque des revendications 6 à 9, comportant en outre un dispositif de détection configuré pour détecter au moins la taille, la position, et/ou l'épaisseur d'au moins une partie du site, ou un dispositif (17) de détection qui est configuré pour détecter au moins la taille, la position, et/ou l'épaisseur d'au moins une partie du site durant une intervention chirurgicale.

11. Système selon la revendication 10, dans lequel les informations provenant du dispositif (17) de détection sont utilisées pour mettre à jour un ensemble d'instructions du robot (13) qui commandent le mouvement du dispositif.

12. Système selon la revendication 10 ou 11, dans lequel les informations provenant du dispositif (17) de détection sont utilisés pour augmenter ou diminuer la pression de fluide administré au site.

13. Système selon l'une quelconque des revendications 10 à 12, dans lequel le dispositif (17) de détection est configuré pour être introduit dans un orifice naturel d'un sujet humain.

14. Système selon l'une quelconque des revendications 10 à 13, dans lequel le dispositif (17) de détection est fixé au dispositif.
